# EUROPÄISCHE PATENTSCHRIFT

(11) **EP 3 349 029 B1**
(45) Veröffentlichungstag und Bekanntmachung des Hinweises auf die Patenterteilung: **11.09.2019**
(21) Anmeldenummer: 17151488.8
(22) Anmeldetag: 13.01.2017
(51) Int. Cl.: G01R 33/28, G01R 33/38, A61B 5/055

(54) **MRT-VORRICHTUNG ZUR VERMESSUNG ZUMINDEST EINES TEILBEREICHS EINES KOPFES**
MAGNETIC RESONANCE TOMOGRAPH FOR MEASURING AT LEAST ONE SECTION OF A HEAD
DISPOSITIF IRM DE MESURE D'AU MOINS UNE ZONE PARTIELLE D'UNE TÊTE

(43) Veröffentlichungstag der Anmeldung: 18.07.2018
(73) Patentinhaber: Sirona Dental Systems GmbH, 64625 Bensheim (DE)
(72) Erfinder: Niederführ, Tilman, 69469 Weinheim (DE); Stöckl, Klaus, 64625 Bensheim (DE); Abkai, Ciamak, 68542 Heddesheim (DE); Scheibl, Guido, 64404 Bickenbach (DE); Ulrici, Johannes, 64285 Darmstadt (DE)
(74) Vertreter: Özer, Alpdeniz

(56) Entgegenhaltungen:
- DE-A1-102009 027 119
- JP-A- 2001 104 278
- US-A- 5 008 624
- US-A1- 2012 324 648
- ZELLER, MARIO; GREISER, ANDREAS: "Utilizing RFID-tags for Planning a Magnetic Resonance Examination", PRIOR ART PUBLISHING, 30. März 2015 (2015-03-30), Seiten 1-2, XP040665517, Germany
- DARROW R.D. ET AL.: "Automatic Landmark for MRI Scanners", PROCEEDINGS OF THE INTERNATIONAL SOCIETY FOR MAGNETIC RESONANCE IN MEDICINE, 20TH ANNUAL MEETING & EXHIBITION, 5. Mai 2012 (2012-05-05), Seite 1451, XP040623874,

## Beschreibung

### Technisches Gebiet

Die Erfindung betrifft eine MRT-Vorrichtung zur Vermessung zumindest eines Teilbereichs eines Kopfes eines Patienten, umfassend mindestens eine Hauptmagnetfeldeinheit und mindestens eine Patientenöffnung.

### Stand der Technik

Aus dem Stand der Technik sind mehrere MRT-Vorrichtungen zur Vermessung eines Patienten bekannt.

DE 10 2009 027119 B4 offenbart ein MRT-System zur bildgebenden Erfassung eines Kopfbereichs mit einem Permanentmagneten, einer Gradientenspule und mindestens einer Hochfrequenzspule, wobei eine Magnetfeldeinheit an einem vertikal angeordneten Stativ in der Höhe verstellt werden kann. Zusätzlich kann die Magnetfeldeinheit bis zu einem Winkel von 45 ° relativ zur Längsachse des Stativs geschwenkt werden. Der Patient nimmt während der Vermessung eine sitzende Position ein, wobei der Kopf des Patienten durch eine Stirnstütze und Ohroliven relativ zum Stativ positioniert wird.

Bei diesem MRT-System wird der Patient auf eine aufwändige Art und Weise relativ zur MRT-Vorrichtung positioniert, indem der Patient in einer zylinderförmigen Patientenöffnung positioniert wird. Dabei werden ein höhenverstellbarer Hocker und das vertikal verstellbare Stativ des MRT-Systems manuell so eingestellt, dass der Kopf des Patienten vermessen werden kann.

DE 197 34 138 B2 offenbart eine MRT-Vorrichtung, umfassend eine Gradienten-Spulenanordnung und eine MRT-Hauptspule.

US 2013/0023418 A1 offenbart eine MRT-Vorrichtung mit einem Kühlungssystem aus einer ersten Stufe und einer zweiten Stufe.

JP 2001104278A offenbart eine MRT-Vorrichtung mit einem Patientenstuhl, wobei ein Patient in sitzender Position auf dem Patientenstuhl positioniert wird und der Patientenstuhl gesteuert durch eine Steuerungseinheit verfahren wird, um den Patienten in die MRT-Vorrichtung zu bringen. Zur Positionierung des Patienten auf dem Patientenstuhl werden mehrere Gürtel verwendet, um den Patienten am Patientenstuhl zu fixieren.

US 2012/324648 A1 offenbart eine PET-Vorrichtung, die auch als eine MRT-Vorrichtung oder CT-Vorrichtung ausgeführt sein kann. Die Vorrichtung zur Vermessung eines Kopfbereiches eines Patienten mit einer schräg angeordneten Mittelachse weist einen Patientensitz auf, der unter Verwendung von mechanischen Verstellmitteln in eine Meßposition hineingefahren werden kann, wobei die Fahrkurve der Verstellmittel parallel zur Mittelachse der MRT-Vorrichtung angeordnet ist.

US 5,008,624 offenbart eine MRT-Vorrichtung mit einem höhenverstellbaren Patientenstuhl und einer verkippbaren Gantry.

Die Aufgabe der vorliegenden Erfindung besteht also darin, eine MRT-Vorrichtung bereitzustellen, die eine bequeme und einfache Positionierung des Patienten relativ zur MRT-Vorrichtung ermöglicht.

### Darstellung der Erfindung

Die Erfindung betrifft eine MRT-Vorrichtung zur Vermessung zumindest eines Teilbereichs eines Kopfes eines Patienten, umfassend mindestens eine Hauptmagnetfeldeinheit und mindestens eine Patientenöffnung. Die Merkmale der MRT-Vorrichtung sind in Anspruch 1 definiert.

Die MRT-Vorrichtung weist einen Patientensitz auf, wobei der Patientensitz Verstellmittel aufweist, die durch mindestens ein Antriebsmittel angetrieben werden, welches mittels einer Steuerungseinheit angesteuert wird, um den Patientensitz in die Patientenöffnung entlang einer Fahrkurve bis zu einer Aufnahmeposition hineinzufahren, in der der Kopf des Patienten zumindest teilweise in einem Aufnahmebereich der MRT-Vorrichtung angeordnet ist. Am Patientensitz ist ein relativ zum Patientensitz verstellbares Element angebracht, wobei mittels einer Detektorvorrichtung an der MRT-Vorrichtung eine Lage des verstellbaren Elements relativ zur Hauptmagnetfeldeinheit und/oder relativ zum Patientensitz automatisch ermittelt wird. Das verstellbare Element ist ein Patientenpositionierungselement und/oder ein bezüglich des positionierten Patienten einzustellendes Element.

Die MRT-Vorrichtung (Magnetresonanztomographie-Vorrichtung) ist eine herkömmliche MRT-Vorrichtung zur Vermessung eines Kopfes. Die MRT-Vorrichtung weist dabei ein Messvolumen auf, wobei das Objektvolumen des Objekts innerhalb des Messvolumens angeordnet wird, um das Objekt, nämlich den Teilbereich des Kopfes zu vermessen.

Die Hauptmagnetfeldeinheit erzeugt das Hauptmagnetfeld und kann beispielsweise aus mindestens einem Dauermagneten, mindestens einer Magnetspule oder mindestens einer supraleitenden Magnetspule bestehen.

Die Patientenöffnung kann beliebig geformt sein und beispielsweise eine kreisförmige Form, eine elliptische Form oder eine rechteckige Form aufweisen. Die Patientenöffnung muss so groß gestaltet sein, dass insbesondere der Kopfbereich des Patienten in den Messbereich der MRT-Vorrichtung hineinpasst.

Der zu vermessende Teilbereich des Kopfes kann beispielsweise einen Unterkiefer, einen Oberkiefer und/oder die Kiefergelenke umfassen.

Der Patientensitz kann eine Rückenlehne und eine Sitzfläche aufweisen, wobei der Patientensitz an der MRT-Vorrichtung angeordnet ist oder eine bekannte Lage relativ zur MRT-Vorrichtung aufweist. Ein Verstellmittel des Patientensitzes kann beispielsweise eine Führungsschiene sein, die den Patientensitz relativ zur MRT-Vorrichtung entlang der Fahrkurve führt. Das Antriebsmittel kann beispielsweise ein Elektromotor, ein hydraulischer Antrieb oder ein pneumatischer Antrieb sein. Die Steuerungseinheit kann beispielsweise ein Mikrocomputer oder ein Mikrochip sein, wobei die Steuerungseinheit das Antriebsmittel so ansteuert, dass der Patientensitz, wie gewünscht, verstellt wird. Beim Positionieren des Patienten wird der Patientensitz in die Patientenöffnung entlang der Fahrkurve bis zu der Aufnahmeposition hineingefahren. Die Aufnahmeposition ist durch den aufzunehmenden Teilbereich des Kopfes bestimmt. Denn in der Aufnahmeposition wird der aufzunehmende Teilbereich des Kopfes innerhalb des Aufnahmebereichs der MRT-Vorrichtung angeordnet. Das verstellbare Element ist am Patientensitz angeordnet und kann manuell durch einen Benutzer oder auch angetrieben durch zweite Verstellmittel und ein zweites Antriebsmittel verstellt werden. Das verstellbare Element wird vor dem Hineinfahren des Patienten relativ zum Patienten positioniert. Die Detektorvorrichtung ist an der MRT-Vorrichtung angeordnet und ermöglicht die Ermittlung der Lage des verstellbaren Elements relativ zur Hauptmagnetfeldeinheit und/oder relativ zum Patientensitz.

Ein Vorteil der vorliegenden MRT-Vorrichtung besteht darin, dass der auf dem Patientensitz positionierte Patient auf eine komfortable Art und Weise vollautomatisch in die MRT-Vorrichtung hineingefahren werden kann. Durch die automatische Ermittlung der Lage des verstellbaren Elements wird indirekt die Lage des Kopfes des Kopfes des Patienten und damit des aufzunehmenden Teilbereichs des Kopfes relativ zur Hauptmagnetfeldeinheit und/oder relativ zum Patientensitz ermittelt, so dass die Länge der noch zu verfahrenen Fahrkurve bis zu der Aufnahmeposition bestimmt werden kann, in der der aufzunehmende Teilbereich des Kopfes im Aufnahmebereich der MRT-Vorrichtung angeordnet ist.

Ein weiterer Vorteil der vorliegenden MRT-Vorrichtung besteht darin, dass durch die Ermittlung der Lage vor dem Hineinfahren oder beim Hineinfahren und durch die automatische Positionierung des Patienten im Vergleich zu einer manuellen Positionierung des Patienten Positionierungsfehler verhindert werden.

Vorteilhafterweise kann das verstellbare Element ein Patientenpositionierungselement, insbesondere eine verstellbare Kinnauflage, eine verstellbare Kopfauflage, verstellbare Ohroliven, verstellbare Kopfhörer und/oder ein bzgl. des positionierten Patienten einzustellendes Element, insbesondere eine verstellbare Hochfrequenzspule, sein.

Das verstellbare Element kann beispielsweise unter Verwendung eines zweiten Verstellmittels, wie einer Führungsschiene, relativ zum Patientensitz, verstellt werden. Die Kinnauflage, die Kopfauflage, die Ohroliven, der Kopfhörer und/oder die Hochfrequenzspule können beispielsweise entlang einer solchen Führungsschiene manuell durch einen Benutzer oder durch ein Antriebsmittel angetrieben relativ zum Patientenstuhl verstellt werden. Das verstellbare Element wird in Abhängigkeit von der Lage des Kopfes relativ zum Patientenstuhl, also der Größe des Patienten, eingestellt. Anschließend erfolgt das Hineinfahren des Patientenstuhls in die MRT-Vorrichtung.

Bei der Ermittlung der Lage des verstellbaren Elements vor dem Hineinfahren des Patienten kann also die Länge der noch zu verfahrenden Fahrkurve bestimmt werden. Bei der Ermittlung der Lage beim Hineinfahren des Patienten kann die Länge eines Restweges bis zum Erreichen der Aufnahmeposition bestimmt werden. Bei der dritten Alternative wird die Lage beim Erreichen der Aufnahmeposition bestimmt, in der das Antriebsmittel angehalten wird und das Hineinfahren des Patientensitzes gestoppt wird.

Vorteilhafterweise kann die Fahrachse einen Winkel relativ zu einer Mittelachse der MRT-Vorrichtung aufweisen, der innerhalb eines Toleranzwinkelbereichs zwischen +10° und -10° relativ zu einer Mittelachse der MRT-Vorrichtung angeordnet ist.

Die Mittelachse der MRT-Vorrichtung kann beispielsweise mit einer Symmetrieachse der Patientenöffnung übereinstimmen. Die Mittelachse der MRT-Vorrichtung kann auch mit der Symmetrieachse des Hauptmagnetfeldes der Hauptmagnetfeldeinheit übereinstimmen bzw. parallel zu der Symmetrieachse des Hauptmagnetfeldes ausgerichtet sein.

Durch das Ausrichten der Fahrkurve bzw. des Verstellmittels, wie einer Führungsschiene, parallel zur Mittelachse der MRT-Vorrichtung wird ein bequemes Hineinfahren des Patientenstuhls in die Patientenöffnung ermöglicht.

Vorteilhafterweise kann die Detektorvorrichtung eine Laser-Vorrichtung sein, die auf einem Triangulation-Messverfahren, einem Phasenverschiebung-Verfahren oder einem Laufzeit-Verfahren beruht, wobei die Laser-Vorrichtung einen Laser, einen Reflektor und einen Sensor umfasst, wobei der Laser eine definierte Lage relativ zur MRT-Vorrichtung aufweist, wobei der Reflektor am Element angeordnet ist.

Durch die Verwendung der Laser-Vorrichtung wird eine präzise Ermittlung der Lage des verstellbaren Elements ermöglicht.

Vorteilhafterweise kann die Detektorvorrichtung eine Seilzug-Vorrichtung umfassend einen Seilzug-Potentiometer und eine Seilöse sein, wobei der Seilzug-Potentiometer eine definierte Lage relativ zur MRT-Vorrichtung aufweist und die Seilöse am Element angeordnet ist.

Durch die Verwendung der Seilzug-Vorrichtung wird eine zuverlässige Ermittlung der Lage des verstellbaren Elements ermöglicht.

Vorteilhafterweise kann die Detektorvorrichtung einen Sensor, wie eine Lichtschranke, und eine Schaltfahne aufweisen, wobei der Sensor eine definierte Lage relativ zur MRT-Vorrichtung aufweist und die Schaltfahne am verstellbaren Element angeordnet ist, wobei der Sensor so angeordnet ist, dass beim Hineinfahren des Patientensitzes oder beim Erreichen der Aufnahmeposition der Sensor ein Signal erzeugt, sobald die Schaltfahne den Sensor durchfährt.

Durch die Verwendung der Lichtschranke wird eine zuverlässige Ermittlung der Lage des verstellbaren Elements beim Hineinfahren des Patientenstuhls ermöglicht. Vorteilhafterweise kann die MRT-Vorrichtung eine Auswahlvorrichtung aufweisen, wobei die einzustellende Aufnahmeposition des Patienten relativ zum Aufnahmebereich der MRT-Vorrichtung mittels der Auswahlvorrichtung in Abhängigkeit von einer aufzunehmenden anatomischen Struktur des Patienten, einer festgelegten Applikation und/oder einer patientenspezifischen Information computergestützt automatisch oder durch einen Benutzer festgelegt wird.

Die Auswahlvorrichtung kann beispielsweise als ein Auswahlmenü mit Bedienungselementen, wie mechanischen Knöpfen oder virtuellen Knöpfen an einem Touchscreen, ausgeführt sein. Die Auswahlvorrichtung ermöglicht beispielsweise die Auswahl einer aufzunehmenden anatomischen Struktur, einer festgelegten Applikation und/oder einer patientenspezifischen Information. Die aufzunehmende anatomische Struktur kann beispielsweise ein Unterkiefer, ein Oberkiefer und/oder Kiefergelenke sein. Die festgelegte Applikation kann beispielsweise eine virtuelle Panoramaschichtaufnahme, eine zephalometrische Aufnahme oder eine 3D-Volumenaufnahme eines bestimmten Bereichs sein. Die patientenspezifischen Informationen können sich beispielsweise auf die Lage des aufzunehmenden Teilbereichs des Kopfes beziehen.

Die Erfindung betrifft weiterhin ein Verfahren gemäss Anspruch 4 zur Positionierung eines Kopfes eines Patienten relativ zu einer MRT-Vorrichtung, umfassend mindestens eine Hauptmagnetfeldeinheit und mindestens eine Patientenöffnung. An der MRT-Vorrichtung ist dabei ein Patientensitz angebracht, wobei der Patientensitz Verstellmittel aufweist, die durch mindestens ein Antriebsmittel angetrieben werden, welches mittels einer Steuerungseinheit angesteuert wird, um den Patientensitz in die Patientenöffnung entlang einer Fahrkurve bis zu einer Aufnahmeposition hineinzufahren, in der der Kopf des Patienten zumindest teilweise in einem Aufnahmebereich der MRT-Vorrichtung angeordnet ist, wobei am Patientensitz ein relativ zum Patientensitz verstellbares Element angebracht ist. Mittels einer Detektorvorrichtung an der MRT-Vorrichtung wird eine Lage des verstellbaren Elements relativ zur Hauptmagnetfeldeinheit und/oder relativ zum Patientensitz automatisch ermittelt, wobei die Lage des verstellbaren Elements verwendet wird, um eine Länge der Fahrkurve des Patientensitzes für die Ansteuerung der Steuerungseinheit zu bestimmen.

Bei dem vorliegenden Verfahren zur Positionierung wird die oben genannte MRT-Vorrichtung verwendet, um den Patienten vollautomisch in die Aufnahmeposition hineinzufahren. Die bestimmte Lage des verstellbaren Elements wird verwendet, um die Länge der zu verfahrenden Fahrkurve bis zum Erreichen der Aufnahmeposition zu ermitteln.

Ein Vorteil dieses Verfahrens besteht darin, dass der Patient auf eine bequeme und präzise Art und Weise automatisch in die Aufnahmeposition hineingefahren wird. Dadurch werden also im Vergleich zum manuellen Positionieren des Patienten Positionierungsfehler verhindert.

Vorteilhafterweise kann die einzustellende Aufnahmeposition des Patienten relativ zum Aufnahmebereich der MRT-Vorrichtung mittels einer Auswahlvorrichtung in Abhängigkeit von einer aufzunehmenden anatomischen Struktur des Patienten, einer festgelegten Applikation und/oder einer patientenspezifischen Information computergestützt automatisch oder manuell durch einen Benutzer festgelegt werden.

Mittels der Auswahlvorrichtung wird also die aufzunehmende anatomische Struktur, die festgelegte Applikation oder die patientenspezifische Information ausgewählt. In Abhängigkeit von dieser Auswahl wird dann die Lage der Aufnahmeposition relativ zum Aufnahmebereich der MRT-Vorrichtung festgelegt.

Die Auswahlvorrichtung kann an der MRT-Vorrichtung oder auch extern außerhalb der MRT-Vorrichtung, beispielsweise in Form eines PCs mit einem Monitor und Bedienungselementen, wie einer Maus und einer Tastatur, angeordnet sein.

Vorteilhafterweise kann in einem ersten Schritt der Patient auf dem Patientensitz positioniert werden, wobei in einem zweiten Schritt das verstellbare Element in Bezug zu einer aufnehmenden anatomischen Struktur des Patienten eingestellt wird, wobei in einem dritten Schritt der auf dem Patientensitz positionierte Patient mittels der Verstellmittel in die Aufnahmeposition verfahren wird.

Durch diesen Ablauf der einzelnen Verfahrensschritte wird eine präzise Positionierung des Patienten gewährleistet.

Vorteilhafterweise kann beim Erreichen der festgelegten Aufnahmeposition das Antriebsmittel automatisch abgeschaltet werden.

Durch das Abschalten des Antriebsmittels mittels der Steuerungseinheit wird also das Hineinfahren des Patientenstuhls gestoppt.

Vorteilhafterweise kann die Detektorvorrichtung die Lage des verstellbaren Elements relativ zur MRT-Vorrichtung vor dem Hineinfahren des Patientensitzes, beim Hineinfahren des Patientensitzes oder erst beim Erreichen der Aufnahmeposition ermitteln.

Bei der ersten Alternative wird die Lage vor dem Hineinfahren des Patientensitzes ermittelt, so dass die Länge der noch zu verfahrenden Fahrkurve bestimmt werden kann. Bei der zweiten Alternative wird die Lage beim Hineinfahren des Patientensitzes ermittelt, so dass ein Restweg bis zum Erreichen der Aufnahmeposition bestimmt werden kann. Bei der dritten Alternative wird die Lage erst beim Erreichen der Aufnahmeposition ermittelt, so dass anschließend das Antriebsmittel abgeschaltet und das Hineinfahren gestoppt wird.

### Kurzbeschreibung der Zeichnungen

Die Erfindung wird anhand der Zeichnungen erläutert. Es zeigt, die
- Fig. 1: eine Skizze einer MRT-Vorrichtung, die
- Fig. 2: die Verstellung der Kopfstütze, die
- Fig. 3: eine Skizze zur Verdeutlichung der Verstellung der Hochfrequenzspule.

### Ausführungsbeispiele

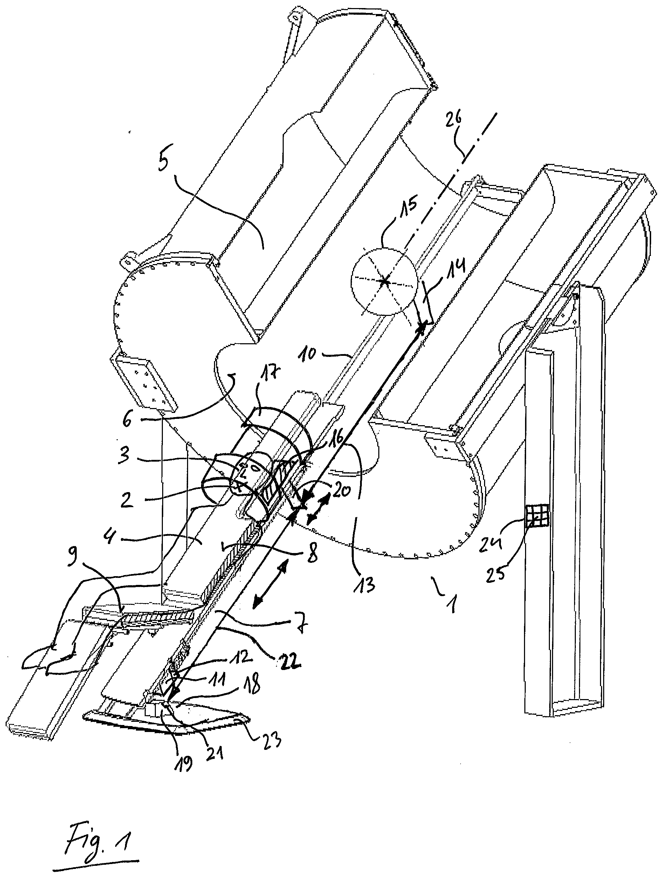

Die Fig. 1 zeigt eine Skizze einer MRT-Vorrichtung 1 zur Vermessung zumindest eines Teilbereichs 2 eines Kopfes 3 eines Patienten 4, umfassend mindestens eine Hauptmagnetfeldeinheit 5 und mindestens eine Patientenöffnung 6. Die MRT-Vorrichtung 1 weist darüber hinaus einen Patientensitz 7 auf, der eine Rückenlehne 8 und eine Sitzfläche 9 aufweist. Darüber hinaus weist der Patientensitz 7 ein Verstellmittel 10, wie eine Führungsschiene, und ein Antriebsmittel 11, wie einen Elektromotor auf, um den Patientensitz in die Patientenöffnung 6 hineinzufahren. Das Antriebsmittel 11 wird dabei mittels einer Steuerungseinheit 12 angesteuert, um den Patientensitz 7 in die Patientenöffnung 6 entlang einer Fahrkurve 13, die als ein Pfeil dargestellt ist, bis zu einer Aufnahmeposition 14 hineinzufahren, in der der Teilbereich 2 des Kopfes 3 in einem Aufnahmebereich 15 der MRT-Vorrichtung angeordnet ist. Der Aufnahmebereich 15 der MRT-Vorrichtung 1 ist im vorliegenden Fall skizzenhaft durch eine Kugel dargestellt. Der aufzunehmende Teilbereich 2 ist skizzenhaft durch ein Kreuz angedeutet. In der Aufnahmeposition 14 ist der aufzunehmende Teilbereich 2 innerhalb des Aufnahmebereichs 15 der MRT-Vorrichtung 1 angeordnet. Am Patientensitz 7 ist ein verstellbares Element, wie eine Kopfauflage 16 und/oder eine Hochfrequenzspule 17 angeordnet, die entlang einer Verstellachse mittels eines zweiten Verstellmittels, wie einer Führungsschiene, relativ zum Patientensitz 7 manuell oder durch ein Antriebsmittel angetrieben verstellbar ist, wie durch den Pfeil angedeutet ist. Die Kopfauflage 16 kann zusammen mit der Hochfrequenzspule 17 oder unabhängig von der Hochfrequenzspule 17 verstellt werden. Mittels einer Detektorvorrichtung 18, die im vorliegenden Fall als eine Laser-Vorrichtung ausgeführt ist und einen Laser 19, ein Reflektor 20 sowie einen Sensor 21 aufweist. Das Laser-Licht des Lasers 19 wird vom Reflektor 20 reflektiert und mittels des Sensors 21 detektiert, so dass ein Abstand 22 zwischen dem Laser 19 und dem Reflektor 20 ermittelt wird. Der Reflektor 20 ist dabei am verstellbaren Element, wie der Kopfauflage 16 und/oder an der Hochfrequenzspule angebracht, so dass die Lage des verstellbaren Elements relativ zum Laser 19 und damit relativ zum Patientensitz 7 und/oder relativ zur Hauptfeldeinheit 5 ermittelt werden kann. Denn der Laser 19 ist an einer definierten Lage relativ zur Hauptfeldeinheit 5 an einem Standfuß 23 angeordnet. Die Detektorvorrichtung 18 kann auch als eine Seilzug-Vorrichtung bestehend aus einem Seilzugpositionierter und einer Seilöse am verstellbaren Element oder als eine Lichtschranke mit einer Schaltfahne am verstellbaren Element 16, 17 ausgeführt sein. Die Schaltfahne kann dabei an einer beliebigen definierten Lage relativ zur Hauptmagnetfeldeinheit 5 angeordnet sein. An der MRT-Vorrichtung 1 ist eine Auswahlvorrichtung 24 mit Auswahlelementen 25, wie Knöpfen oder virtuellen Bedienungselementen auf einem Touchscreen, ausgeführt. Die Auswahlvorrichtung 24 ermöglicht die Auswahl einer aufzunehmenden anatomischen Struktur, einer festgelegten Applikation und/oder einer patientenspezifischen Information. Die Aufnahmeposition 14 ist durch die Lage des Reflektors 20 dem Hineinfahren des Patientensitzes 7 entlang der Fahrkurve 13 angedeutet, wobei der Patient sowie Patientensitz 7 in der Aufnahmeposition 14 aus Gründen der Übersichtlichkeit nicht dargestellt wurden. Die Fahrachse 13, die durch die Schiene 10 bestimmt wird, ist dabei parallel oder zumindest innerhalb eines Toleranzbereichs zwischen + 10° und - 10° relativ zu einer Mittelachse 26 der MRT-Vorrichtung 1 angeordnet. Die Mittelachse 26 stimmt im vorliegenden Fall mit einer Symmetrieachse der Patientenöffnung 6 und mit einer Symmetrieachse eines Hauptmagnetfeldes der Hauptmagnetfeldeinheit 5 überein.

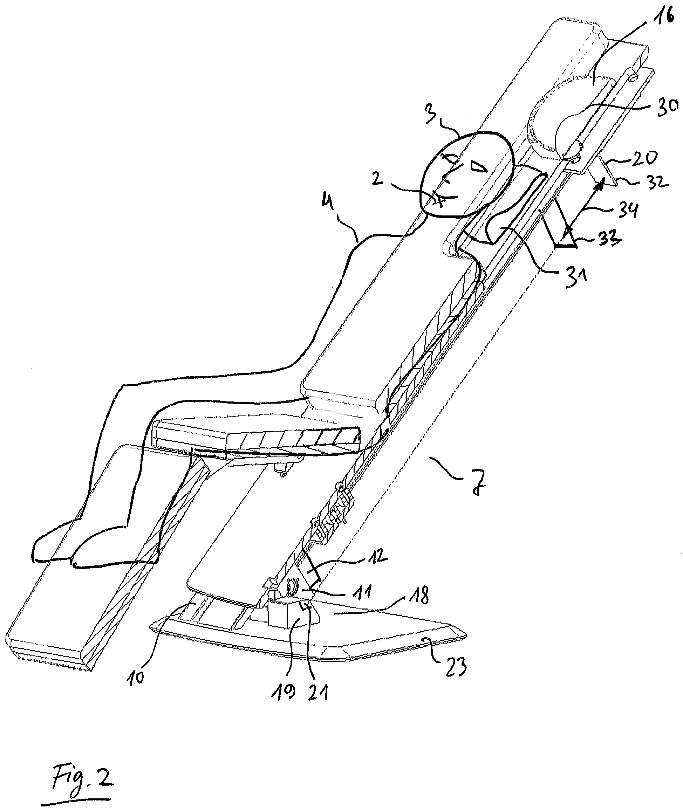

Die Fig. 2 zeigt die Verstellung der Kopfstütze 16 relativ zum Patientensitz 7 ausgehend von einer Ausgangsposition 30 in einer Endposition 31, in der die Kopfhalterung 16 unterhalb des Kopfes 3 des Patienten 4 angeordnet wird. Bei der Verschiebung der Kopfhalterung 16 wird auch der Reflektor 20 ausgehend von einer Ausgangsposition 32 in eine Endposition 33 verschoben, wie durch den Pfeil 34 angedeutet ist. Die Verschiebung der Kopfhalterung 16 kann beispielsweise mittels eines zweiten Verstellmittels, wie einer nicht dargestellten Führungsschiene, erfolgen.

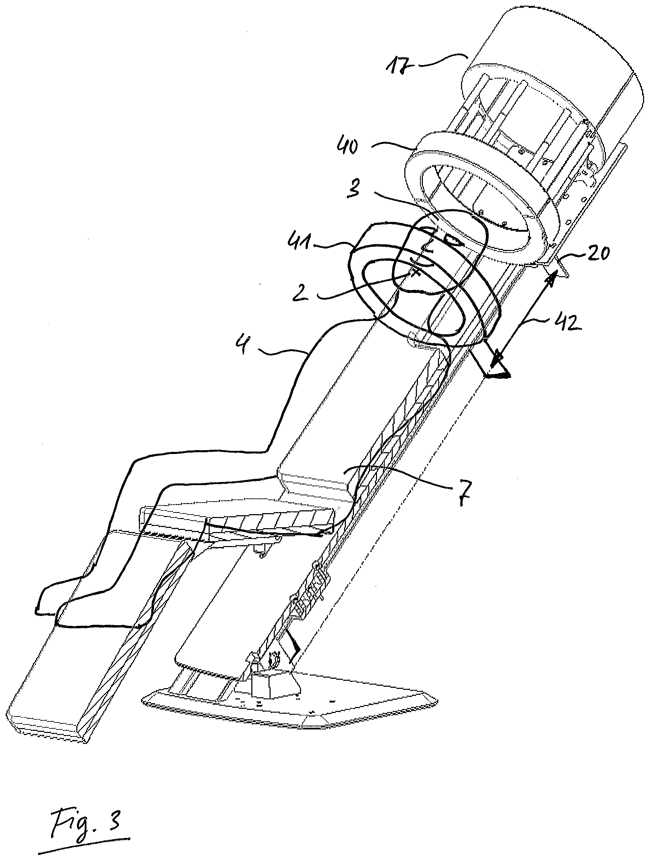

Die Fig. 3 zeigt eine Skizze zur Verdeutlichung der Verstellung der Hochfrequenzspule 17 relativ zum Patientenstuhl 7 ausgehend von einer Ausgangsposition 40 in eine Endposition 41, wie durch den Pfeil 42 angedeutet ist. Die Hochfrequenzspule 17 ist in der Endposition 41 nur durch den unteren Ring angeordnet, so dass der aufzunehmende Teilbereich 2 des Kopfes 3 des Patienten 4 innerhalb der Hochfrequenzspule angeordnet ist.

Nach dem Verstellen des verstellbaren Elements, wie der Kopfhalterung 16 aus Fig. 2 oder der Hochfrequenzspule 17 aus Fig. 3 wird der Patientensitz 7 in die Patientenöffnung 6 bis zur Aufnahmeposition 14 hineingefahren, wie in Fig. 1 dargestellt ist.

### Bezugszeichen

- 1: MRT-Vorrichtung
- 2: Teilbereich
- 3: Kopf
- 4: Patient
- 5: Hauptmagnetfeldeinheit
- 6: Patientenöffnung
- 7: Patientensitz
- 8: Rückenlehne
- 9: Sitzfläche
- 10: Verstellmittel
- 11: Antriebsmittel
- 12: Steuerungseinheit
- 13: Fahrkurve
- 14: Aufnahmeposition
- 15: Aufnahmebereich
- 16: Kopfauflage
- 17: Hochfrequenzspule
- 18: Detektorvorrichtung
- 19: Laser
- 20: Reflektor
- 21: Sensor
- 22: Abstand
- 23: Standfuß
- 24: Auswahlvorrichtung
- 25: Auswahlelemente
- 26: Mittelachse
- 30: Ausgangsposition
- 31: Endposition
- 32: Ausgangsposition
- 33: Endposition
- 34: Pfeil
- 40: Ausgangsposition
- 41: Endposition
- 42: Pfeil

## Patentansprüche

1. MRT-Vorrichtung (1) zur Vermessung zumindest eines Teilbereichs (2) eines Kopfes (3) eines Patienten (4), umfassend mindestens eine Hauptmagnetfeldeinheit (5) und mindestens eine Patientenöffnung (6), wobei die MRT-Vorrichtung (1) einen Patientensitz (7) und eine Steuerungseinheit (12) aufweist, wobei der Patientensitz (7) ein Verstellmittel (10) und mindestens ein Antriebsmittel (11) aufweist, wobei das Verstellmittel (10) durch das Antriebsmittel (11) angetrieben wird, wobei die Steuerungseinheit (12) dazu eingerichtet ist, das Antriebsmittel (11) anzusteuern, um den Patientensitz (7) in die Patientenöffnung (6) entlang einer Fahrkurve (13) bis zu einer Aufnahmeposition (14) hineinzufahren, in der der Kopf (3) des Patienten (4) zumindest teilweise in einem Aufnahmebereich (15) der MRT-Vorrichtung (1) angeordnet ist, wobei die MRT-Vorrichtung (1) zusätzlich ein verstellbares Element (16, 17) aufweist, das am Patientensitz (7) angebracht ist und relativ zum Patientensitz (7) verstellbar ist, **dadurch gekennzeichnet, dass** die MRT-Vorrichtung (1) zusätzlich eine Detektorvorrichtung (18) aufweist, wobei mittels der Detektorvorrichtung (18) eine Lage des verstellbaren Elements (16, 17) relativ zur Hauptmagnetfeldeinheit (5) und/oder relativ zum Patientensitz (7) automatisch ermittelbar ist, um eine Länge der Fahrkurve (13) für die Ansteuerung der Steuerungseinheit (12) zu bestimmen, wobei das verstellbare Element (16, 17) ein Patientenpositionierungselement und/oder ein bezüglich des positionierten Patienten einzustellendes Element ist.

2. MRT-Vorrichtung (1) nach Anspruch 1, **dadurch gekennzeichnet, dass** das verstellbare Element (16, 17) ein Patientenpositionierungselement, insbesondere eine verstellbare Kinnauflage, eine verstellbare Kopfauflage (16), verstellbare Ohroliven, verstellbare Kopfhörer und/oder ein bzgl. des positionierten Patienten einzustellendes Element, insbesondere eine verstellbare Hochfrequenzspule (17), ist.

3. MRT-Vorrichtung (1) nach Anspruch 1 oder 2, **dadurch gekennzeichnet, dass** die MRT-Vorrichtung (1) eine Auswahlvorrichtung (24) aufweist, wobei die einzustellende Aufnahmeposition (14) des Patienten relativ zum Aufnahmebereich (15) der MRT-Vorrichtung (1) mittels der Auswahlvorrichtung (24) in Abhängigkeit von einer aufzunehmenden anatomischen Struktur des Patienten, einer festgelegten Applikation und/oder einer patientenspezifischen Information computergestützt automatisch oder durch einen Benutzer festlegbar ist.

4. Verfahren zur Positionierung eines Kopfes (3) eines Patienten (4) relativ zu einer MRT-Vorrichtung (1), umfassend mindestens eine Hauptmagnetfeldeinheit (5) und mindestens eine Patientenöffnung (6), wobei an der MRT-Vorrichtung (1) ein Patientensitz (7) angebracht ist, wobei der Patientensitz (7) Verstellmittel (10) aufweist, die durch mindestens ein Antriebsmittel (11) angetrieben werden, welches mittels einer Steuerungseinheit (12) angesteuert wird, um den Patientensitz (7) in die Patientenöffnung (6) entlang einer Fahrkurve (13) bis zu einer Aufnahmeposition (14) hineinzufahren, in der der Kopf (3) des Patienten (4) zumindest teilweise in einem Aufnahmebereich (15) der MRT-Vorrichtung (1) angeordnet ist, wobei am Patientensitz (7) ein relativ zum Patientensitz (7) verstellbares Element (16, 17) angebracht ist, **dadurch gekennzeichnet, dass** mittels einer Detektorvorrichtung (18) an der MRT-Vorrichtung (1) eine Lage des verstellbaren Elements (16, 17) relativ zur Hauptmagnetfeldeinheit (5) und/oder relativ zum Patientensitz (7) automatisch ermittelt wird, wobei die Lage des verstellbaren Elements (16, 17) verwendet wird, um eine Länge der Fahrkurve (13) des Patientensitzes (7) für die Ansteuerung der Steuerungseinheit (12) zu bestimmen.

5. Verfahren nach Anspruch 4, **dadurch gekennzeichnet, dass** die einzustellende Aufnahmeposition (14) des Patienten (4) relativ zum Aufnahmebereich (15) der MRT-Vorrichtung (1) mittels einer Auswahlvorrichtung (24) in Abhängigkeit von einer aufzunehmenden anatomischen Struktur des Patienten, einer festgelegten Applikation und/oder einer patientenspezifischen Information computergestützt automatisch oder manuell durch einen Benutzer festgelegt wird.

6. Verfahren nach Anspruch 4 oder 5, **dadurch gekennzeichnet, dass** in einem ersten Schritt der Patient (4) auf dem Patientensitz (7) positioniert wird, wobei in einem zweiten Schritt das verstellbare Element (16, 17) in Bezug zu einer aufnehmenden anatomischen Struktur des Patienten (4) eingestellt wird, wobei in einem dritten Schritt der auf dem Patientensitz (7) positionierte Patient (4) mittels der Verstellmittel (10) in die Aufnahmeposition (14) verfahren wird.

7. Verfahren nach einem der Ansprüche 4 bis 6, **dadurch gekennzeichnet, dass** beim Erreichen der festgelegten Aufnahmeposition (14) das Antriebsmittel (11) automatisch abgeschaltet wird.

8. Verfahren nach einem der Ansprüche 4 bis 7, **dadurch gekennzeichnet, dass** die Detektorvorrichtung (18) die Lage des verstellbaren Elements (16, 17) relativ zur MRT-Vorrichtung (1) vor dem Hineinfahren des Patientensitzes (7), beim Hineinfahren des Patientensitzes (7) oder erst beim Erreichen der Aufnahmeposition (14) ermittelt.

## Claims

1. MRI machine (1) for measuring at least a portion (2) of a head (3) of a patient (4), comprising at least one primary magnetic field unit (5) and at least one patient opening (6), wherein the MRI machine (1) comprises a patient seat (7) and a control unit (12), wherein the patient seat (7) comprises adjusting means (10) and at least one drive means (11), wherein the adjusting means (10) is driven by the drive means (11), wherein the control unit (12) is configured to control the drive means (11) in order to move the patient seat (7) into the patient opening (6) along a travel curve (13) as far as an imaging position (14), in which the head (3) of the patient (4) is at least partially positioned within an imaging region (15) of the MRI machine (1), wherein the MRI machine (1) additionally comprises an adjustable element (16, 17) which is attached to the patient seat (7) and is adjustable relative to said patient seat (7), **characterized in that** the MRI machine (1) additionally comprises a detector apparatus (18), wherein a position of the adjustable element (16, 17) relative to the primary magnetic field unit (5) and/or relative to the patient seat (7) can be identified automatically with the aid of the detector apparatus (18) in order to determine a length of the travel curve (13) for the control of the control unit (12), wherein the adjustable element (16, 17) is a patient positioning element and/or an element that can be adjusted relative to the positioned patient.

2. MRI machine (1) according to Claim 1, **characterized in that** the adjustable element (16, 17) is a patient positioning element, in particular a chin rest, an adjustable headrest (16), adjustable earpieces, adjustable head phones and/or an element that can be adjusted relative to the positioned patient, in particular an adjustable high-frequency coil (17).

3. MRI machine (1) according to Claim 1 or 2, **characterized in that** the MRI machine (1) comprises a selection apparatus (24), wherein the imaging position (14) of the patient to be adjusted relative to the imaging region (15) of the MRI machine (1) can be determined automatically with computer assistance or by a user using the selection apparatus (24) as a function of an anatomical structure of the patient to be imaged, a specified application and/or patient-specific information.

4. Method for positioning a head (3) of a patient (4) relative to an MRI machine (1) comprising at least one primary magnetic field unit (5) and at least one patient opening (6), wherein a patient seat (7) is attached to the MRI machine (1), wherein the patient seat (7) comprises adjusting means (10) which are driven by at least one drive means (11) that is controlled by means of a control unit (12) in order to move the patient seat (7) into the patient opening (6) along a travel curve (13) as far as an imaging position (14), in which the head (3) of the patient (4) is at least partially positioned within an imaging region (15) of the MRI machine (1), wherein an adjustable element (16, 17), which is adjustable relative to the patient seat (7), is attached to said patient seat (7), **characterized in that** a position of the adjustable element (16, 17) relative to the primary magnetic field unit (5) and/or relative to the patient seat (7) is identified automatically with the aid of a detector apparatus (18) on the MRI machine (1), wherein the position of the adjustable element (16, 17) is used to determine a length of the travel curve (13) of the patient seat (7) for the control of the control unit (12) .

5. Method according to Claim 4, **characterized in that** the imaging position (14) of the patient (4) to be adjusted relative to the imaging region (15) of the MRI machine (1) is determined automatically with computer assistance or manually by a user using a selection apparatus (24) as a function of an anatomical structure of the patient to be imaged, a specified application and/or patient-specific information.

6. Method according to Claim 4 or 5, **characterized in that**, in a first step, the patient (4) is positioned on the patient seat (7), wherein, in a second step, the adjustable element (16, 17) is adjusted relative to an anatomical structure of the patient (4) to be imaged, wherein, in a third step, the patient (4) positioned on the patient seat (7) is moved into the imaging position (14) by means of the adjusting means (10).

7. Method according to any one of Claims 4 to 6, **characterized in that** the drive means (11) is automatically switched off when the determined imaging position (14) has been reached.

8. Method according to any one of Claims 4 to 7, **characterized in that** the detector apparatus (18) determines the position of the adjustable element (16, 17) relative to the MRI machine (1) before moving the patient seat (7) in, when the patient seat (7) is moved in, or only upon reaching the imaging position (14).

## Revendications

1. Dispositif d'IRM (1) destiné à la mesure d'au moins une zone partielle (2) de la tête (3) d'un patient (4), comprenant au moins une unité de champ magnétique principale (5) et au moins une ouverture pour patient (6), ledit dispositif d'IRM (1) présentant un siège pour patient (7) et une unité de commande (12), le siège pour patient (7) présentant un moyen de réglage (10) et au moins un moyen d'entraînement (11), le moyen de réglage (10) étant entraîné par le moyen d'entraînement (11), l'unité de commande (12) étant conçue pour commander le moyen d'entraînement (11) afin d'introduire le siège pour patient (7) dans l'ouverture pour patient (6) le long d'une courbe de déplacement (13) jusqu'à une position d'imagerie (14) dans laquelle la tête (3) du patient (4) est au moins partiellement disposée dans une zone d'imagerie (15) du dispositif d'IRM (1), ledit dispositif d'IRM (1) présentant en outre un élément réglable (16, 17) qui est agencé sur le siège pour patient (7) et qui est réglable par rapport au siège pour patient (7), **caractérisé en ce que** le dispositif d'IRM (1) présente en outre un dispositif détecteur (18) au moyen duquel dispositif détecteur (18) une position de l'élément réglable (16, 17) peut être détectée automatiquement par rapport à l'unité de champ magnétique principale (5) et/ou par rapport au siège pour patient (7) afin de déterminer une longueur de la courbe de déplacement (13) pour la commande de l'unité de commande (12) ; l'élément réglable (16, 17) étant un élément de positionnement de patient et/ou un élément à régler par rapport au patient positionné.

2. Dispositif d'IRM (1) selon la revendication 1, **caractérisé en ce que** l'élément réglable (16, 17) est un élément de positionnement de patient, notamment un support pour menton réglable, un support de tête réglable (16), des embouts auriculaires réglables, des écouteurs réglables et/ou un élément à régler par rapport au patient positionné, notamment un bobinage haute fréquence réglable (17).

3. Dispositif d'IRM (1) selon la revendication 1 ou 2, **caractérisé en ce que** le dispositif d'IRM (1) présente un dispositif de sélection (24), la position d'imagerie (14) du patient à régler par rapport à la zone d'imagerie (15) du dispositif d'IRM (1) pouvant être fixée de façon automatisée par voie informatique ou par un utilisateur au moyen du dispositif de sélection (24) en fonction d'une structure anatomique à imager du patient, d'une application définie et/ou d'une information spécifique du patient.

4. Procédé de positionnement de la tête (3) d'un patient (4) par rapport à un dispositif d'IRM (1), comprenant au moins une unité de champ magnétique principale (5) et au moins une ouverture pour patient (6), un siège pour patient (7) étant disposé sur le dispositif d'IRM (1), le siège pour patient (7) présentant des moyens de réglage (10) qui sont entraînés par au moins un moyen d'entraînement (11) commandé au moyen d'une unité de commande (12) afin d'introduire le siège pour patient (7) dans l'ouverture pour patient (6) le long d'une courbe de déplacement (13) jusqu'à une position d'imagerie (14) dans laquelle la tête (3) du patient (4) est au moins partiellement disposée dans une zone d'imagerie (15) du dispositif d'IRM (1), un élément réglable (16, 17) par rapport au siège pour patient (7) étant disposé sur le siège pour patient (7), **caractérisé en ce que,** au moyen d'un dispositif détecteur (18) présent sur le dispositif d'IRM (1), une position de l'élément réglable (16, 17) par rapport à l'unité de champ magnétique principale (5) et/ou par rapport au siège pour patient (7) peut être déterminée automatiquement, la position de l'élément réglable (16, 17) servant à déterminer une longueur de la courbe de déplacement (13) du siège pour patient (7) pour la commande de l'unité de commande (12).

5. Procédé selon la revendication 4, **caractérisé en ce que** la position d'imagerie (14) du patient (4) à régler par rapport à la zone d'imagerie (15) du dispositif d'IRM (1) est fixée de façon automatisée par voie informatique ou manuellement par un utilisateur au moyen d'un dispositif de sélection (24) en fonction d'une structure anatomique à imager du patient, d'une application définie et/ou d'une information spécifique du patient.

6. Procédé selon la revendication 4 ou 5, **caractérisé en ce que** pendant une première étape, le patient (4) est positionné sur le siège pour patient (7), pendant une deuxième étape, l'élément réglable (16, 17) est réglé par rapport à une structure anatomique à imager du patient (4), pendant une troisième étape, le patient (4) positionné sur le siège pour patient (7) est déplacé au moyen du moyen de réglage (10) vers la position d'imagerie (14).

7. Procédé selon l'une des revendications 4 à 6, **caractérisé en ce que** lorsque la position d'imagerie (14) définie est atteinte, le moyen d'entraînement (11) est automatiquement éteint.

8. Procédé selon l'une des revendications 4 à 7, **caractérisé en ce que** le dispositif détecteur (18) détermine la position de l'élément réglable (16, 17) par rapport au dispositif d'IRM (1) avant l'introduction du siège pour patient (7), lors de l'introduction du siège pour patient (7) ou seulement lorsque la position d'imagerie (14) est atteinte.
